# EUROPEAN PATENT SPECIFICATION

(11) **EP 2 313 424 B1**
(45) Date of publication and mention of the grant of the patent: **23.04.2014**
(21) Application number: 09775815.5
(22) Date of filing: 09.07.2009
(51) Int. Cl.: C07J 7/00, C07J 31/00, A61K 31/58, A61K 31/57

(54) **ANIONIC PREGNANE COMPOUNDS, METHOD FOR THEIR PRODUCING AND USE OF THEM**
ANIONISCHE PREGNANVERBINDUNGEN, VERFAHREN ZU IHRER HERSTELLUNG UND DEREN ANWENDUNG
COMPOSÉS ANIONIQUES DE PRÉGNANE, LEUR PROCÉDÉ DE FABRICATION ET LEUR UTILISATION

(30) Priority: 10.07.2008 CZ 20080434
(43) Date of publication of application: 27.04.2011
(73) Proprietor: Ústav organické chemie a biochemie Akademie ved Ceské republiky, v.v.i., 166 10 Praha 6 (CZ); Fyziologicky ústav Akademie ved Ceské republiky, v.v.i., 142 20 Praha 4 (CZ)
(72) Inventor: STASTNA, Eva, 750 02 Prerov I (CZ); CHODOUNSKA, Hana, 160 00 Praha 6 (CZ); POUZAR, Vladimir, 140 00 Praha 4 (CZ); KAPRAS, Vojtech, 170 OO Praha 7 (CZ); CAIS, Ondrich, 160 00 Praha 6 (CZ); VYKLICKY, Ladislav, 251 68 Kamenice (CZ); KOHOUT, Ladislav, 163 00 Praha 6 (CZ)
(74) Representative: Herman, Vaclav
(86) International application number: PCT/CZ2009/000091
(87) International publication number: WO 2010/003391

(56) References cited:
- WO-A2-01/60375
- JOURNAL OF PHARMACOLOGY AND EXPERIMENTAL THERAPEUTICS 200006 US, vol. 293, no. 3, June 2000 (2000-06), pages 747-754, XP002563723 ISSN: 0022-3565 cited in the application
- PARK-CHUNG M ET AL: "DISTINCT SITES FOR INVERSE MODULATION OF N-METHYL-D-ASPARTATE RECEPTORS BY SULFATED STEROIDS" MOLECULAR PHARMACOLOGY, AMERICAN SOCIETY FOR PHARMACOLOGY AND EXPERIMENTAL THERAPEUTICS, BALTIMORE, MD, US, vol. 52, no. 6, 1 January 1997 (1997-01-01), pages 1113-1123, XP000911767 ISSN: 0026-895X
- YAU, JOYCE L. W. ET AL: "Central administration of a cytochrome P450-7B product 7.alpha.-hydroxypregnenolone improves spatial memory retention in cognitively impaired aged rats" JOURNAL OF NEUROSCIENCE , 26(43), 11034-11040 CODEN: JNRSDS; ISSN: 0270-6474, 2006, XP002563724
- STASTNA E ET AL: "Synthesis of C3, C5, and C7 pregnane derivatives and their effect on NMDA receptor responses in cultured rat hippocampal neurons" STEROIDS, ELSEVIER SCIENCE PUBLISHERS, NEW YORK, NY, US, vol. 74, no. 2, 24 November 2008 (2008-11-24), pages 256-263, XP025894826 ISSN: 0039-128X [retrieved on 2008-11-24]

## Description

### Field of the Invention

The invention relates to anionic pregnane derivatives and their production. Further, it concerns pharmaceuticals containing these anionic steroid compounds and their use as a neuroprotective against excitotoxic damage of the central nervous system (CNS), conditions associated with excessive activation of NMDA-subtype glutamate receptors or, where this type of receptor is involved in the development or course of several mental and neurological diseases. This is essentially a traumatic and hypoxic damage of nervous tissue in the central nervous system diseases like Alzheimer's, Huntington's, and Parkinson's disease as well as in cognitive disorders arising in old age. Other indications could be tardive dyskinesia, amyotrophic lateral sclerosis, olivopontocerebellar degeneration, neurological problems associated with AIDS infection, allergic encephalomyelitis, and a medication for epilepsy, anxiety, depression, schizophrenia, chronic pain, and drug addiction.

### Background of the Invention

Neurons are part of a system that adequately responds to various stimulations in order to protect the stability of the organism. An indispensable part of the activity of neurons is usually excitation, but under certain conditions may be excessive excitation of neurons harmful. Such a condition known as excitotoxicity, can lead to symptoms characteristic for severe diseases of nervous system, such as Alzheimer's, Huntington's, or Parkinson's disease. These diseases are becoming increasingly serious and growing problem especially for the aging population. Drugs for prevention and care for the sick are urgently needed. One of the goals, where you can intervene against the previously mentioned diseases, is NMDA receptor. It is an ion channel activated by glutamic acid, presented in all areas of the brain and spinal cord.

Medication for pathological conditions, such as craniocerebral injury, stroke and other neurological conditions associated with excitotoxicity, is currently the subject of intensive research. So far, there are no reliable drugs or unambiguously designated active substance that could be applied as a universal medicine. In 2006, the 3rd phase of clinical research for NXY-059 was an unsuccessfully terminated (disodium disufentone; Chacon M.R., Jensen M.B., Sattin J.A., Zivin J.A.: Neuroprotection in cerebral ischemia: emphasis of SAINT trial, Curr. Cardiol. Rep, 2008, 10, 37-42.). NXY was promising substance to improve chances of survival and reduce the consequences of stroke. For the treatment of early stages of Alzheimer's disease is used memantine with questionable therapeutic utility. The utilization of substances that have shown very good neuroprotective effects at excitotoxicity caused by excessive activation of NMDA receptors, both *in vitro* tests and in animal studies, such as drug is prevented by their side effects, in some cases serious mental disorders have been observed (psychosis are common). This group is represented by e.g. ketamine.

Neurosteroids are synthesized in the nervous tissue from cholesterol and/or modified to neuroactive compounds from circulating precursors (Baulieu EE. Neurosteroids: A novel function of the brain. Psychoneuroendocrinology 1998; 23, 963-87.). They act through neuronal membrane receptors for essential transmitters (e.g. γ-amino-butyric acid (GABA) an *N*-methyl-D-aspartate (NMDA)). Such neurosteroids have been proposed to control neuronal excitability by modulating ligand and/or voltage-gated ion channels (Paul SM, Purdy RH. Neuroactive steroids. Faseb J 1992; 6, 2311-22; Biggio G, Concas A, Costa E. Steroid modulation of amino acid neurotransmitter receptors. v: Farb DH, Gibbs TT, Wu FS, Gyenes M, Friedman L, Russek SJ. GABAergic Synaptic Transmission: Molecular, Pharmacological, and Clinical Aspects, New York: Raven Press; 1999, 119-131.) and this action has been shown to affect a lot of physiological processes e.g. learning (Tsien JZ, Huerta PT, Tonegawa S. The essential role of hippocampal CA1 NMDA receptor-dependent synaptic plasticity in spatial memory (Cell 1996; 87, 1327-38.), aging (Vallee M, Mayo W, Darnaudery M, Corpechot C, Young J, Koehl M, et al. Neurosteroids: deficient cognitive performance in aged rats depends on low pregnenolone sulfate levels in the hippocampus. Proc Natl Acad Sci USA 1997; 94, 14865-70.), and stress (Grobin AC, Roth HR, Deutch AY. Regulation of the prefrontal cortical dopamine system by the neuroactive steroid 3α,21-dihydroxy-5α-pregnane-20-one. Brain Res 1992; 578, 351-56.) as well as certain neurological and psychiatric disorders (Alzheimer's disease (Nasman B, Olsson T, Backstrom T, Eriksson S, Grankvist K, Viitanen M, et al. Serum dehydroepiandrosterone sulfate in Alzheimer's disease and in multi-infarct dementia. Biol Psychiatry 1991; 30, 684-90.), epilepsy (Gasior M, Carter RB, Witkin JM. Neuroactive steroids: potential therapeutic use in neurological and psychiatrical disorders. Trends in Pharmacol Sci 1999; 20, 107-112.) etc.).

Defining a binding site of neurosteroids and the knowledge of the molecular mechanism present a molecular template for design and development of novel therapeutic entities. The molecular mechanism by which neurosteroids affect ligand-gated ion channels is still not clearly understood. In general, neurosteroids influence the frequency of single channel openings and the average channel open duration (Twyman RE, Macdonald RL. Neurosteroid regulation of GABA(A) receptor single-channel kinetic-properties of mouse spinal-cord neurons in culture. J Physiol 1992; 456, 215-45; Callachan H, Cottrell GA, Hather NY, Lambert JJ, Nooney JM, Peters JA. Modulation of the GABA-A receptor by progesterone metabolites. Proc R Soc Lond B Biol Sci 1987; 231, 359-369.

Similarly, much attention has been devoted to recognition of the corresponding binding sites and some of them were identified for the GABA_{A} receptor, but not for NMDA receptor. The results of current studies indicate that neurosteroids have their specific binding sites, independent of particular agonists or other allosteric modulators (Park-Chung M, Wu FS, Purdy RH, Malayev AA, Gibbs TT, Farb DH. Distinct sites for inverse modulation of N-methyl-D-aspartate receptors by sulfated steroids. Mol Pharmacol 1997; 52, 1113-23; Akk G, Bracamontes JR, Covey DF, Evers A, Dao T, Steinbach JH. Neuroactive steroids have multiple actions to potentiate GABAA receptors J Physiol 2004; 558, 59-74.).

The knowledge of NMDA receptor's binding site is not as all-embracing as for the GABA receptor; nevertheless, the effect of neurosteroids on NMDA receptor seems to be mediated by independent binding sites located at the extracellular domain of the NMDA receptor (Park-Chung M, Wu FS, Farb DH. 3α-Hydroxy-5β-pregnan-20-one sulfate: a negative modulator of the NDMA-induced current in cultured neurons. Mol Pharmacol 1994; 46,1 46-50; Horak M, Vlcek K, Chodounska H, Vyklicky L. Subtype-dependence of N-methyl-D-aspartate receptor modulation by pregnenolone sulfate. Neuroscience 2006; 137, 93-102.). The experiments with chimeric receptors have shown crucial role of an extracellular loop between the third and fourth transmembrane domains of the NR2 subunit in the mechanism of potentiating and inhibitory effects of 3β-hydroxy-pregn-5-en-20-one and 20-oxo-5β-pregnan-3α-yl sulfate (Horak M, Vlcek K, Chodounska H, Vyklicky L. Subtype-dependence of N-methyl-D-aspartate receptor modulation by pregnenolone sulfate. Neuroscience 2006; 137,93-102; Petrovic M, Sedlacek M, Horak, Chodounska H, Vyklicky L. 20-Oxo-5β-pregnan-3α-yl sulfate is a use-dependent NMDA receptor inhibitor. J Neurosci 2005; 25, 8439-50.).

Without doubt, a lot of other aspects of the receptor structure will influence the nature and extent of the neurosteroid modulation: the stereochemistry of the neurosteroids should be mentioned. Previous structure-activity studies have reported several features which are important for activity of neurosteroids at NMDA receptors: the structure should include a sulfonyloxy group at the position 3 and a 20-oxo group as well (Cais O, Vyklicky L. Psychiatrie Suppl 2006; 10, 8-11.). The potentiating effect is maintained if the sulfate group is replaced by another negatively charged group, e.g. hemioxylate, hemisuccinate or hemiglutarate (Park-Chung M, Wu FS, Purdy RH, Malayev AA, Gibbs, TT, Farb DH. Distinct sites for inverse modulation of N-methyl-D-aspartate receptors by sulfated steroids. Mol Pharmacol 1997; 52, 1113-23; Weaver CE, Land MB, Purdy RH, Richards KG, Gibbs TT, Farb DH. Geometry and charge determine pharmacological effects of steroids on N-methyl-D-aspartate receptor-induced Ca(2+) accumulation and cell death. J Pharniacol Exp Ther 2000; 293, 747-54.).

A number of preclinical studies demonstrate a significant ability of NMDA antagonists to prevent out flux of glutamate and thus limit the disturbance of function of CNS. However, their neuroprotective potential is small from a clinical point of view. Due to the fact that NMDA receptors are one of the most spread type of receptors in CNS, medication with NMDA antagonists leads to a number of serious undesirable effects from the disruption of basic motoric up to the induction of schizophrenic psychosis. On the other hand, different subunit composition of NMDA receptors on presynaptic and postsynaptic elements, on different types of nerve cells and even in different parts of the brain offer the ability to selectively search for substances affecting only a certain subset of NMDA receptors, and thereby reduce the incidence of unexpected and undesirable effects while preserving neuroprotective action.

Steroid derivatives are not yet used in medical practice for medication, although their neuroactivity has been long known and even alfaxolone was removed from medication. The aim of the authors of the present invention are neuroactive anionic compounds which have a high index of selectivity and efficiency, i.e. are less toxic but more effective than previously known analogue.

Therefore, the authors of present invention began development and testing of new NMDA antagonists that are derived from neurosteroids. During this study found that newly synthesized compounds show affinity for extrasynaptic NMDA receptors. What are even more important, electrophysiological studies have shown that this type of substance binds only to long-term open NMDA receptors. Consequently, blocking of excess calcium influx into the cell through long open-NMDA receptors was estimated as neuroprotective mechanism of action.

During these studies were synthesized new pregnane derivatives, which inhibit the NMDA receptor and may therefore be useful for treating CNS diseases, such as cognitive disorders in aging, Alzheimer's disease, stroke, Parkinson's disease, Huntington's disease and other diseases and disorders resulting from excessive activation of NMDA receptors.

### Summary of the Invention

The object of the invention is new pregnane anionic compounds of general formula I 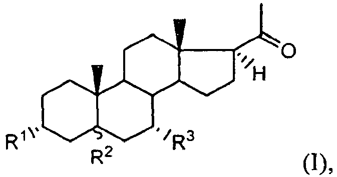 in which
R¹ is the ester group of general formula R⁴O-, which is able to form anion, where R⁴ is HSO₃-, pyridine-SO₃-, HOOC-(CH₂)₂-CO- etc.,
R² is hydrogen atom with the configuration of the alpha or beta and
R³ is ester group of general formula R⁵COO-, where R⁵ is CH₃- or C₅H₄N-, such as acetoxy group or nicotinyloxy group,
and their pharmaceutically acceptable salts.

This invention also includes methods of production of compounds of general formula I in which R¹, R² and R³ represent, as seen above, according to which the compound of formula II 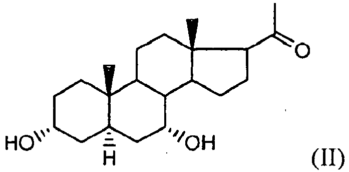 is transferred to 3,7-diprotected derivative, advantageously of diacetate of fomula **III** 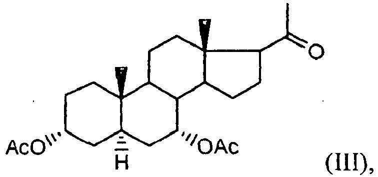 that is then by partial hydrolysis, advantageously of by alkali metal hydroxide, such as potassium, sodium, or lithium hydroxide, or acids such as hydrochloric or perchloric acid etc., in a suitable solvent such as alcohol, aqueous alcohol, or solvent mixtures, advantageously of a mixture of methanol and benzene, converted to monoester formula IV 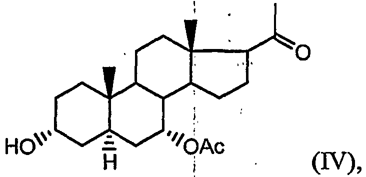 from which by the treatment with succmic anhydride in pyridine in the presence of DMAP can be prepared particular ester, advantageously of hemisuccinate of formula **I** 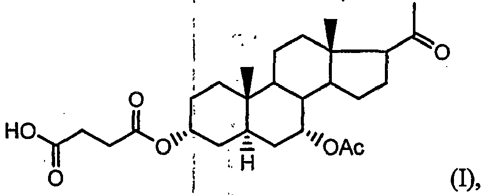 in which R¹ is hemisuccinate group, R² means a hydrogen atom in the alpha position and R³ is acetoxy group,
or by the reaction of pyridine-sulfur trioxide complex in an inert solvent, advantageously in chloroform, can be prepared pyridinium sulfate of general formula **I** 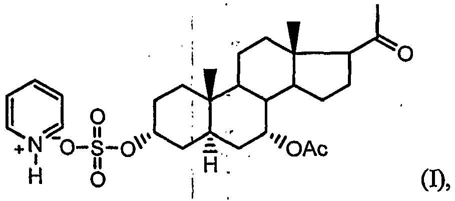 in which R¹ is pyridinium sulfate group, R² means a hydrogen atom in the alpha position and R³ is acetoxy group. Alternatively, the latter compound the formula I 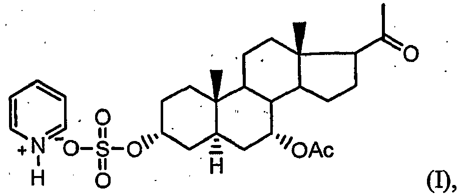 in which R¹ is pyridinium sulfate group, R² means a hydrogen atom in the alpha position and R³ is acetoxy group, can be converted by treatment with sodium hydroxide, in alcohol, advantageously in methanol, to the compound of general formula **I** 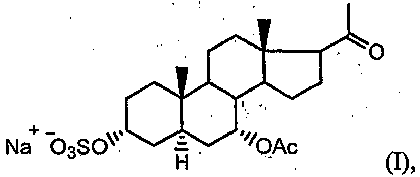 in which R¹ is sodium salt of sulfate, R² is hydrogen atom in the alpha position and R³ is acetoxy group.

Compound of general formule **I** in which R¹ is pyridinium sulfate, R² is hydrogen atom in the alpha position and R³ is nicotinyloxy group, shall be synthesized so that the compound of general formula **IV** 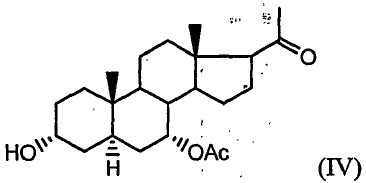 is converted by a reaction with *tert*-butyldimethylsilyl chloride and imidazole in a suitable solvent, advantageously in DMF, to the *tert*-butyldimethylsilyl derivative of formula **V** 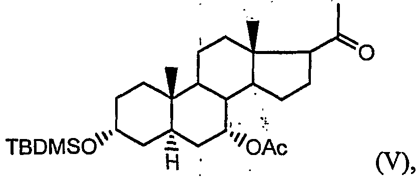 which by the usual saponification of acetate group in position 7, advantageously in potassium hydroxide in a mixture of ethanol and benzene at increased temperature, afforded a compound of formula **VI** 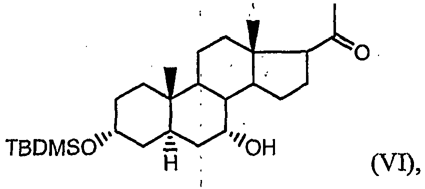 which can be esterified by treatment with nicotinoyl chloride in pyridine in the presence of DMAP to afford nicotinoyloxy derivative of formula **VII** 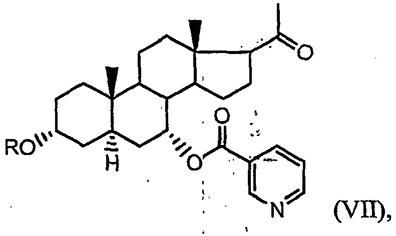 where R is TBDMS, and by the treatment with p-toluenesulfonic acid in methanol provided the compound of general formula **VII** in which R means a hydrogen atom, which then by reaction with pyridine-sulfur trioxide complex in a suitable inert solvent, advantageously in chloroform, afforded the compound of general formula I 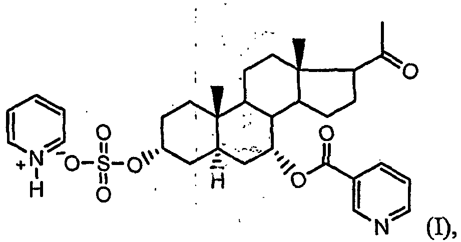 in which R¹ is sodium salt of pyridinium sulfate, R² is hydrogen atom in the alpha position and R³ is nicotinyloxy group.

A similar sequence of reactions can produce compounds of general formula **I** in which R² is hydrogen atom with the beta configuration, from compound of formula **VIII** 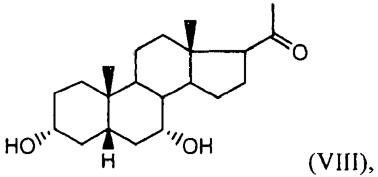 which differs from a compound of formula **II** only in configuration of the hydrogen atoms in position 5.

A similar sequence of reactions, as explained above, allows producing the following compounds of general formula **I:**
20-Oxo-5α-pregnane-3α,7α-diyl 3-Hemisuccinate 7-Acetate,
20-Oxo-5α-pregnane-3α,7α-diyl 3-Sulfate 7-Acetate Pyridinium Salt,
20-Oxo-5α-pregnane-3α,7α-diyl 3-Sulfate 7-Acetate Sodium Salt,
20-Oxo-5α-pregnane-3α,7α-diyl 3-Sulfate 7-Nicotinate Pyridinium Salt,
20-Oxo-5β-pregnane-3α,7α-diyl 3-Hemisuccinate 7-Acetate,
20-Oxo-5β-pregnane-3α,7α-diyl 3-Sulfate 7-Acetate Pyridinium Salt,
20-Oxo-5β-pregnane-3α,7α-diyl 3-Sulfate 7-Acetate Sodium Salt, and
20-Oxo-5β-pregnane-3α,7α-diyl 3-Sulfate 7-Nicotinate Pyridinium Salt.

The object of this invention is the utilization of compounds of general formula I as neuroprotective agents in traumatic brain injury, spinal cord, stroke, in the central nervous system diseases such as Alzheimer's, Huntington's and Parkinson's disease, also in the prevention of CNS disease, and for medication of conditions associated with excitotoxicity. Indications comprise the prevention or deceleration of onset of neurodegeneration in Alzheimer's disease, the consequences of stroke, traumatic brain damage, Parkinson's disease, tardive dyskinesia, Huntington's disease, amyotrophic lateral sclerosis, olivopontocerebellar degeneration, AIDS, allergic encephalomyelitis, and for medication of epilepsy, anxiety, depression, schizophrenia, chronic pain and drug addiction.

The objects of the invention are also pharmaceuticals which as active ingredients include the above compounds of general formula **I**, in the R¹, R² and R³ represent, as seen above, or their substituted analogues, either alone or in combination with any pharmaceutically acceptable carrier, diluents or adjuvant.

Pregnanolone sulfate and its homologs show a positive role in different models of human diseases, such as NMDA-induced epileptic seizures, formalin induced pain and neuroprotective effect, both *in vitro* and *in vivo* models. Based on our findings (see details in the example 20), we believe that homologues ofpregnanolone sulfate may have therapeutic value in the treatment of the central nervous system diseases of a human.

The following examples serve only to illustrate the objects referred in the invention, without thereby in any way limiting this invention, which is given only to the extent of accompanying patent claims.

### EXAMPLES

### Example 1

### 20-Oxo-5α-pregnane-3α,7α-diyl Diacetate

To a solution of 20-oxo-5α-pregnane-3α,7α-diol (100 mg, 0.3 mmol) in pyridine (5 mL), acetic anhydride (0.42 mL, 4.4 mmol) was added and the mixture was heated for 6 h at 50 °C. After standing at room temperature for 2 days, the reaction mixture was poured into ice-water and extracted with ethyl acetate (50 mL). Extract was washed with solution of potassium hydrogen carbonate, water, and dried. Solvents were evaporated and the oily residue purified by preparative thin layer chromatography in a mixture of acetone/petroleum ether (1:1) to give title diacetate (67 mg, 54%): m.p. 112-114 °C, [α]_{D} +21.8 (c 0.363, CHCl₃). IR spectrum (CHCl₃): 1726 (C=O, acetate); 1702 (C=O, ketone); 1259, 1241, 1030 (C-O). ¹H NMR (200 MHz): 0.60 (s, 3 H, 3 × H-18); 0.80 (s, 3 H, 3 × H-19); 2.05 (s, 3 H, C(3)-OAc); 2.08 (s, 3 H, C(7)-OAc); 2.12 (s, 3 H, 3 × H-21); 2.54 (t, 1 H, J = 8.8, H-17); 4.92 (q, 1 H, J = 2.9, H-7); 5.03 (m, 1 H, H-3). FAB MS: 399 (12%, M + Na), 359 (8%, M + 1 - H₂O) 299 (80%, M + 1 - H₂O AcOH). C₂₅H₃₈O₅: Calculated C, 71.7.4; H, 9.15. Found C, 72.44; H, 9.22.

### Example 2

### 3a-Hydroxy-20-oxo-5α-pregnan-7α-yl Acetate

A solution of diacetate from Example 1 (90 mg, 0.22 mmol) in benzene (10 mL) was treated with a solution of potassium hydroxide (15.4 mg, 0.27 mmol) in methanol (1 mL). After standing overnight at room temperature, the mixture was poured into water and extracted with ethyl acetate (50 mL). The extract was washed with water, dried and the solvent was evaporated. The residue was purified by preparative thin layer chromatography in a mixture of acetone/petroleum ether (1:1) to give title compound (69 mg, 85%): m.p. 148-150 °C (ether/petroleum ether), [α]_{D} +64.5 (c 0.346, CHCl₃). IR spectrum (CHCl₃): 3616 (O-H); 1717 (C=O, acetate); 1701 (C=O, ketone); 1256 (C-O). ¹H NMR (200 MHz): 0.59 (s, 3 H, 3 × H-18); 0.78 (s, 3 H, 3 × H-19); 2.07 (s, 3 H, OAc); 2.11 (s, 3 H, 3 × H-21); 2.55 (t, 1 H, J = 8.8, H-17); 4.06 (quintet, 1 H, J = 2.4, H-3); 4.91 (q, 1 H, J = 2.9, H-7). FAB MS: 399 (17%, M + Na), 317 (8%, M + 1 - AcOH), 299 (9%, M + 1- AcOH, H₂O). C₂₃H₃₆O₄: Calculated C, 73.37; H, 9.64. Found C, 74.12; H, 9.87.

### Example 3

### 20-Oxo-5α-pregnane-3α,7α-diyl 3-Hemisuccinate 7-Acetate

A mixture of compound prepared according to Example 2 (100 mg, 0.27 mmol) and succinic anhydride (100 mg, 2 mmol) was dried in vacuo (25 °C, 100 Pa) for 30 min. Dry pyridine (10 mL) and 4-dimethylaminopyridine (20 mg, 0.17 mmol) were added. The mixture was heated for 6 h at 140 °C. Additional succinic anhydride (200 mg, 4 mmol) and 4-dimethylaminopyridine (20 mg, 0.17 mmol) were added and the mixture was heated for 10 h at 140 °C. The reaction mixture was poured into water and extracted with ethyl acetate (50 mL). Aqueous phase was extracted again with ethyl acetate (50 mL), and the collected organic extracts were extracted with water and dried. The solvent was evaporated and the residue crystallized from hot ethyl acetate to give title hemisuccinate (71 mg, 35%): m.p. 193-195 °C, [α]_{D} +43.3 (c 0.246, CHCl₃). IR spectrum (CHCl₃): 3516, 3100 broad (COOH); 1717 (C=O, acetate); 1701 (C=O, ketone); 1379 (CH₃); 1257, 1248 (C-O, acetate). ¹H NMR (200 MHz): 0.60 (s, 3 H, 3 × H-18); 0.80 (s, 3 H, 3 × H-19); 2.08 (s, 3 H, OAc); 2.12 (s, 3 H, 3 × H-21); 2.54 (t, 3 H, J = 8.8, H-17); 2.60-2.71 (m, 4 H, OOCCH₂CH₂COO); 4.92 (m, 1 H, H-3); 5.06 (q, 1 H, J = 2.4, H-7). FAB MS: 499 (100%, M + Na), 417 (13%, M + 1 - AcOH), 299 (55%, M - AcOH, C₄H₅O₄). HR-MS (+ESI) Calculated for C₂₇H₄₀O₇Na [M⁺] 499.2666, found 499.2662. C₂₇H₄₀O₇: Calculated C, 68.04; H, 8.46. Found C, 67.83; H, 8.46.

### Example 4

### 20-Oxo-5α-pregnane-3α,7α-diyl 3-Sulfate 7-Acetate Pyridinium Salt

The mixture of compound prepared according to Example 2 (200 mg, 0.53 mmol) and a sulfur trioxide pyridine complex (400 mg, 2.5 mmol), dried in vacuo (25 °C, 100 Pa) for 1 h, was dissolved in dry chloroform (10 mL). The reaction mixture was stirred for 4 h at room temperature under argon. After standing overnight at -20 °C, the undissolved sulfur trioxide pyridine complex was filtered off The solvent was evaporated and the residue was dissolved in absolute methanol (1 mL) with diethylether (15 mL). The mixture was concentrated to almost one-half. After standing overnight at -20 °C, the crystals were collected, filtered off and dried in a desiccator (over potassium hydroxide) to afford compound 6 (230 mg, 82%): m.p. 156-160 °C, [α]_{D} +29.3 (c 0.288, CHCl₃). IR spectrum (CHCl₃): 3072 (pyridinium); 1716 (C=O, acetate); 1702 (C=O, ketone); 1258, 1220 (C-O acetate); 1258 (S-O). ¹H NMR (400 MHz): 0.59 (s, 3 H, 3 × H-18); 0.80 (s, 3 H, 3 ×-H-19); 2.06 (s, 3 H, OAc); 2.12 (s, 3 H, 3 × H-21); 2.56 (t, 1 H, J = 9.2, H-17); 4.78 (quintet, 1 H, J = 2.6, H-3); 4.89 (q, 1 H, J = 2.9, H-7); 7.98 (m, 2 H, H-3 and H-5, pyridinium); 8.48 (tt, 1 H, J₁ = 7.8, J₂ =1.5, H-4, pyridinium); 8.94 (m, 2 H, H-2 and H-6, pyridinium). ESI MS: 574 (17%, M + K), 494 (14%, M + K - pyridinium), 478 (21%, M + Na-pyridinium), 412 (15%, M - CH₃CO, pyridinium), 341 (47%, M + Na - OSO₃C₅H₆N, CH₃CO). HR-MS (+ESI) Calculated for C₂₈H₄₁O₇NNaS [M⁺ + Na] 558.2496, found 558.2502. C₂₈H₄₁NO₇S: Calculated C, 62.78; H, 7.71; N, 2.61; S, 5.99. Found C, 62.38; H, 7.62; N, 2.70; S, 6.73.

### Example 5

### 20-Oxo-5α-pregnan-3α,7α-diyl 3-Sulfate 7-Acetate Sodium Salt

The compound prepared according to Example 4 (140 mg, 16.1 mmol) was dissolved in absolute methanol (2 mL) and solution of sodium hydroxide in methanol (0.9 mL, 0.373 M) was added dropwise during 1 h to pH 8. The solution was concentrated to one-half and absolute ether (10 mL) was added. After standing overnight at -20 °C, the obtained crystals were filtered off and dried in a desiccator (over potassium hydroxide) to yield title sodium salt (86 mg, 68%): m.p. 162-163 °C, [α]_{D} +29.6 (c 0.215, CHCl₃). IR spectrum (CHCl₃): 1733, 1717 (C=O, acetate); 1703 (C=O, ketone); 1257, 1245, 1198 (C-O, acetate). ¹H NMR (200 MHz): 0.61 (s, 3 H, 3 × H-18); 0.79 (s, 3 H, 3 × H-19); 2.10 (s, 3 H, OAc); 2.11 (s, 3 H, 3 × H-21); 2.54 (t, 1 H, J = 8.8, H-17); 4.69 (m, 1 H, H-3); 4.85 (m, 1 H, H-7). ESI MS: 501 (12%, M + Na), 381 (100%, M + Na - OSO₃Na). C₂₃H₃₅NaO₇S + 2H₂O: Calculated C, 53.68; H, 7.63; S, 6.23. Found C, 53.52; H, 7.64; S, 6.11.

### Example 6

### 3α-(tert-Butyldimethylsilyloxy)-20-oxo-5α-pregnan-7α-yl Acetate

Hydroxy derivative prepared in Example 2 (528 mg, 1.4 mmol) and imidazole (570 mg, 8.37 mmol) were dissolved in N,N-dimethylformamide (11 mL) and the mixture was cooled to 0 °C. Then, *tert*-butyldimethylsilyl chloride was added (528 mg, 3.5 mmol). The reaction mixture was allowed to attain room temperature and stirred. After 2 h, the reaction was diluted with ethyl acetate (150 mL), organic phase was washed with solution of citric acid, potassium hydrogen carbonate, and water. The organic layer was dried and evaporated in vacuo. Crystallization from ethyl acetate gave 687 mg (99%) of title compound: m.p. 120-121 °C, [α]_{D} +29.0 (c 0.369, CHCl₃). IR spectrum (CHCl₃): 2897 ((CH₃)₂Si); 1715 (C=O, acetate); 1701 (C=O, ketone); 1472, 1463 ((CH₃)₃C); 1258 (C-O, acetate); 1053 (C-OSi). ¹H NMR (200 MHz): 0.02 (s, 6 H, 6 × (CH₃)₂Si); 0.59 (s, 3 H, 3 × H-18); 0.76 (s, 3 H, 3 × H-19); 0.89 (s, 9 H, (CH₃)₃C); 2.03 (s, 3 H, OAc); 2.12 (s, 3 H, 3 × H-21); 2.54 (t, 1 H, J = 8.7, H-17); 3.97 (quintet, 1 H, J = 2.9, H-3); 4.89 (q, 1 H, J = 2.4, H-7). ESI MS: 513 (83%, M + Na), 457 (20%, M + Na - (CH₃)₃C), 353 (42%, M + Na - (CH₃)₃C(CH₃)₂SiO, CH₃CO, AcOH). C₂₉H₅₀O₄Si: Calculated C, 70.97; H, 10.27. Found C, 70.49; H, 10.39.

### Example 7

### 3α-(tert-Butyldimethylsilyloxy)-7α-hydroxy-5α-pregnan-20-one

The solution of acetate prepared; in Example 6 (650 mg, 1.32 mmol) in benzene (50 mL) and potassium hydroxide in ethanol (0.89 M, 60 mL) were heated at 60 °C for 16 h. The mixture was poured into water and extracted with ethyl acetate (80 mL). The organic layer was washed with water, dried and the solvents were evaporated in vacuo. The residue was crystallized from hot ethyl acetate to give title compound (527 mg, 88%): m.p. 135-140 °C (ethyl acetate), [α]_{D} +63.3 (c 0.232, CHCl₃). IR spectrum (CHCl₃): 3615 (O-H); 1699 (C=O, ketone); 1472, 1463 ((CH₃)₃C); 1253 ((CH₃)₂Si); 1052 (C-OSi). ¹H NMR (200 MHz): 0.02 (s, 6 H, (CH₃)₂Si); 0.59 (s, 3 H, 3 × H-18); 0.88 (s, 12 H, 3 × H-19 and (CH₃)₃C); 2.11 (s, 3 H, 3 × H-21); 2.56 (t, 1 H, J = 8.8, H-17); 3.83 (m, 1 H, H-7); 3.98 (m, 1 H, H-3). ESI MS: 919 (100%, 2M + Na), 471 (50%, M + Na). C₂₇H₄₈O₃Si: Calculated C, 72.26; H, 10.70. Found C, 72.25; H, 11.11.

### Example 8

### 3α-(tert-Butyldimethylsilyloxy)-20-oxo-5α-pregnan-7α-yl Nicotinate

Compound prepared in previous Example 7 (450 mg, 1.0 mmol) and 4-dimethylaminopyridine (10 mg, 0.09 mmol) were dissolved in pyridine (15 mL) and the solution was cooled to 0 °C. Nicotinoyl chloride hydrochlorile (900 mg, 5.0 mmol) was added to a stirred mixture in small portions. The reaction mixture was stirred at room temperature for 3 h. Then, it was poured into water (50 mL) and, after standing 15 h at 5 °C, the precipitate of title product was separated by suction and subsequently dried in a desiccator (over potassium hydroxide) overnight to yield title compound (498 mg, 89%): m.p. 147-151 °C, [α]_{D} +14.5 (c 0.391, CHCl₃). IR spectrum (CHCl₃): 2897 ((CH₃)₂Si); 1714 (C=O, nicotinate); 1703 (C=O, ketone); 1286 (C-O, nicotinate); 1053 (C-OSi). ¹H NMR (400 MHz): 0.05 (s, 6 H, (CH₃)₂Si); 0.64 (s, 3 H, 3 × H-18); 0.72 (s, 9 H, (CH₃)₃C); 0.83 (s, 3 H, 3 × H-19); 2.11 (s, 3 H, 3 × H-21); 2.51 (t, 1 H, J = 8.8, H-17); 3.96 (quintet, J = 2.4, 1 H, H-3); 5.22 (q, 1 H, J = 2.6, H-7); 7.40 (ddd, 1 H, J₁ = 7.8, J₂ = 4.8, J₃ = 0.7, H-5, nicotinate); 8.29 (dt, 1 H, J₁ = 8, J₂ = 2, H-4, nicotinate); 8.79 (dd, 1 H, J₁ = 4.8, J₂ = 1.8, H-6, nicotinate); 9.25 (m, 1 H, H-₂, nicotinate). FAB MS: 554 (36%, M + 1), 496 (8%, M - (CH₃)₃C), 299 (4%, M - (CH₃)₃C(CH₃)₂SiO, OCOC₅H₄N), 255 (79%, M - (CH₃)₃(CH₃)₂SiO, OCOC₅H₄N, CH₃CO). C₃₃H₅₁NO₄Si: calculated C, 71.56; H, 9.28; N, 2.53. Found C, 71.40; H, 9.41; N, 2.25.

### Example 9

### 3α-Hydroxy-20-oxo-5α-pregnan-7α-yl Nicotinate

Compound prepared in previous Example 8 (120 mg, 0.21 mmol) was dissolved in methanolic solution of p-toluenesulfonic acid (0.005 M, 72 mL). After standing at room temperature for 10 days, the mixture was neutralized with 10% potassium carbonate solution, extracted with ethyl acetate (100 mL), organic layer was washed with water and dried. The solvents were evaporated and the product was purified by preparative thin layer chromatography in a mixture of petroleum ether/acetone (8:2) to give title compound (83 mg, 87%): m.p. 183-187°C (acetone/heptane), [α]_{D} +15.0 (c 0.169, CHCl₃). IR spectrum (CHCl₃): 3615 (O-H); 1715 (C=O, nicotinate); 1703 (C=O, ketone); 1287 (C-O, nicotinate); 1002 (C-OH). ¹H NMR (200 MHz): 0.72 (s, 3 H, 3 × H-18); 0.86 (s, 3 H, 3 × H-19); 2.25 (s, 3 H, 3 × H-21); 2.53 (t, 1 H, J = 8.8, H-17); 4.05 (quintet, 1 H, J = 2.4, H-3); 5.25 (q, 1 H, J = 2.8, H-7); 7.44 (m, 1 H, H-5, nicotinate); 8.30 (dt, 1 H, J₁ = 7.8, J₂ = 1.9, H-4, nicotinate); 8.78 (dd, 1 H, J₁ = 4.8, J₂ = 1.4, H-6, nicotinate); 9.26 (m, 1 H, H-2, nicotinate). ESI MS: 901 (100%, 2M + Na), 462 (93%, M + Na), 440 (24%). C₂₇H₃₇NO₄: Calculated C, 73.77; H, 8.48; N, 3.19. Found C, 73.75; H, 9.24; N, 2.53.

### Example 10

### 20-Oxo-5α-pregnane-3α,7α-diyl 3-Sulfate 7-Nicotinate Pyridinium Salt

The mixture of compound prepared in previous Example 9 (60 mg, 0.13 mmol) and a sulfur trioxide pyridine complex (120 mg, 0.75 mmol), dried in vacuo (25 °C, 100 Pa) for 1 h, was dissolved in dry chloroform (5 mL). The reaction mixture was stirred for 4 h at room temperature under argon. After standing overnight at -20 °C, the undissolved sulfur trioxide pyridine complex was filtered off. The solvents were evaporated and the residue was dissolved in absolute methanol (1 ml) with added diethyl ether (5 ml). The mixture was evaporated to almost one-half. After standing overnight at -20 °C, the solvents were evaporated to afford title product (71 mg, 87%) as a foam: [α]_{D} +6.0 (c 0.204, CHCl₃). IR spectrum (CHCl₃): 3457, 3139 (pyridinium); 1715 (C=O, nicotinate); 1702 (C=O, ketone); 1289 (C-O, nicotinate); 1243, 1046 (SO₃). ¹H NMR (400 MHz): 0.63 (s, 3 H, 3 × H-18); 0.87 (s, 3 H, 3 × H-19); 2.11 (s, 3 H, 3 × H-21); 2.54 (t, 1 H, J = 8.7, H-17); 4.79 (quintet, J = 2.7, 1 H, H-3); 5.25 (q, 1 H, J = 2.5, H-7); 7.74 (dd, 1 H, J₁ = 7.8, J₂ = 5.5, H-5, nicotinate); 7.83 (m, 2 H, H-3 and H-5, pyridinium); 8.30 (tt, 1 H, J₁ = 7.8, J₂ = 1.5, H-4, pyridinium); 8.62 (dt, 1 H, J₁ = 7.8, J₂ = 1.5, H-4, nicotinate); 8.84 (m, 2 H, H-2 and H-6, pyridinium); 8.93 (m, 1 H, H-6, nicotinate); 9.29 (m, 1 H, H-2, nicotinate). FAB MS: 554 (3%, M - CH₃CO), 440 (0.5%, M - 2 × C₅H₆N), 422 (10%, M + 1 - OSO₃C₅H₆N). HR-MS (+ESI) Calculated for C₃₂H₄₂O₇N₂NaS [M⁺ + Na] 621.2605, found 621.2609. C₃₂H₄₂N₂O₇S: Calculated C, 64.19; H, 7.07; N, 4.68; S, 5.36. Found C, 60.87; H, 7.33; N, 3.55; S, 5.37.

### Example 11

### 3α-Hydroxy-20-oxo-5β-pregnan-7α-yl Acetate

A solution of 20-oxo-5β-pregnane-3α,7α-diyl diacetate (100 mg, 0.24 mmol) known from previous art in methanol (8 mL) was treated with a solution of potassium hydrogen carbonate in water (0.7 mL, 0.2 M) at 70 °C. After 5 h, the mixture was poured into water and extracted with ethyl acetate (70 mL). The organic layer was washed with water, dried and the solvents were evaporated. Crystallization afforded title compound (40 mg, 45%): m.p. 143-145 °C, [α]_{D} +40.3 (c 0.303, CHCl₃). IR spectrum (CHCl₃): 3610, 3527 (O-H); 1726 (C=O, acetate); 1703 (C=O, ketone); 1252 (C-O, acetate); 1047 (C-O). ¹H NMR (200 MHz): 0.61 (s, 3 H, 3 × H-18); 0.93 (s, 3 H, 3 × H-19); 2.06 (s, 3 H, OAc); 2.12 (s, 3 H, 3 × H-21); 2.55 (t, 1 H, J = 8.8, H-17); 3.52 (m, 1 H, W = 32.7, H-3); 4.89 (q, 1 H, J = 2.4, H-7). FAB MS: 377 (5%, M + 1), 317 (12%, M + 1 - AcOH), 299 (40%, M + 1 - AcOH, H₂O), 283 (32%, M - AcOH, H₂O, CH₃), 255 (17%, M - AcOH, H₂O, CH₃CO), 159 (30%), 145 (34%), 131 (33%), 119 (37%). C₂₃H₃₆O₄: Calculated C, 73.37; H, 9.64. Found C, 73.49; H, 9.93.

### Example 12

### 20-Oxo-5β-pregnane-3α,7α-diyl 3-Hemisuccinate 7-Acetate

A mixture of title compound prepared in previous Example 11 (100 mg, 0.27 mmol) and succinic anhydride (200 mg, 2 mmol) was dried in vacuo (25 °C, 100 Pa) for 30 min. Dry pyridine (20 mL) and 4-dimethylaminopyridine (20 mg, 0.17 mmol) were added. The mixture was heated for 4 h at 140 °C. The reaction mixture Was poured into water and extracted with ethyl acetate (50 mL). Aqueous phase was extradted again with ethyl acetate (50 mL), washed with water and dried. The residue by preparative thin layer chromatography in a mixture of petroleum ether/acetone (9:1) afforded the title compound (44 mg, 35%): m.p. 131-134 °C, [α]_{D} +50.3 (c 0.218, CHCl₃). IR spectrum (CHCl₃): 3517, 2676 broad (COOH); 1756 (C=O, COOH); 1726 (C=O, acetate); 1720 (C=O, hemisuccinate); 1703 (C=O, ketone); 1252 (C-O, acetate); 1173 (C-O, hemisuccinate). ¹H NMR (200 MHz): 0.61 (s, 3 H, 3 x H-18); 0.94 (s, 3 H, 3 x H-19); 2.06 (s, 3 H, OAc); 2.12 (s, 3 H, 3 x H-21); 2.54-2.69 (m, 5 H, H-17 and OOCCH₂CH₂COO); 4.62 (m, 1 H, W = 31.8, H-3); 4.89 (q, 1 H, J = 2.7, H-7). FAB MS: 499 (69%, M + Na), 417 (18%, M + 1-AcOH), 299 (28%, M - AcOH, C₄H₅O₄). HR-MS (+ESI) Calculated for C₂₇H₄₀O₇Na [M⁺ + Na] 499.2666, found 499.2664. C₂₇H₄₀O₇: Calculated C, 68.04; H, 8.46. Found C, 69.20; H, 8.59.

### Example 13

### 20-Oxo-5β-pregnan-3α,7α-diyl 3-Sulfate 7-Acetate Pyridinium Salt

The mixture of compound prepared in Example 11 (110 mg, 0.3 mmol) and a sulfur trioxide pyridine complex (220 mg, 1.4 mmol), dried in vacuo (25 °C, 100 Pa) for 1 h, was dissolved in dry chloroform (4 mL). The reaction mixture was stirred under argon at room temperature for 4 h. After standing overnight at -20 °C, the undissolved sulfur trioxide pyridine complex was filtered off. The solvent was evaporated and the residue was dissolved in absolute methanol (1 mL) and absolute ether (7 mL) was added. The mixture was concentrated to almost one-half of volume. After standing overnight at -20 °C, the crystals of title compound were collected and dried in a desiccator (over potassium hydroxide) (155 mg, 99%): m.p. 155-158 °C, [α]_{D} + 45.7 (c 0.285, CHCl₃). IR spectrum (CHCl₃): 3140 (pyridinium); 1725 (C=O, acetate); 1702 (C=O, ketone); 1253 (C-O); 1363, 1171, 960 (O-SO₃). ¹H NMR (200 MHz): 0.60 (s, 3 H, 3 x H-18); 0.92 (s, 3 H, 3 x H-19); 2.04 (s, 3 H, OAc); 2.56 (t, 1 H, J = 8.7, H-17); 4.34 (m, 1 H, W = 31, H-3); 4.88 (q, 1 H, J = 2.9, H-7); 7.97 (m, 2 H, H-3 and H-5, pyridinium); 8.48 (tt, 1 H, J₁ = 7.8, J₂ = 1.5, H-4, pyridinium); 8.95 (m, 2 H, H-2 and H-6, pyridinium). EI MS: 558 (0.5 %, M + Na), 455 (0.5%, M - pyridinium), 256 (0.5%, M - pyridinium, OSO₃, CH₃CO, AcOH), 230 (3%), 80 (100%). HR-MS (+ESI) Calculated for C₂₈H₄₁O₇NNaS [M⁺ + Na] 558.2496, found 558.2501. C₂₈H₄₁NO₇S Calculated C, 62.78; H, 7.71; N, 2.61; S, 5.99. Found C, 59.99; H, 7.71; N, 2.61; S, 6.25.

### Example 14

### 20-Oxo-5β-pregnane-3α,7α-diyl 3-Sulfate 7-Acetate Sodium Salt

Pyridinium salt prepared in Example 13 (90 mg, 0.17 mmol) was dissolved in absolute methanol (2 mL) and a solution of sodium hydroxide in methanol (0.45 mL, 0.373 M) was added dropwise during 1 h up to pH 8. The solution was concentrated to one-half and absolute ether (6 mL) was added. After standing overnight at -20 °C, the obtained crystals were collected and dried in a desiccator (over potassium hydroxide) to give title compound (54 mg, 67%): m.p. 187-191 °C, [α]_{D} + 35.0 (c 0.248, CHCl₃). IR spectrum (CHCl₃): 1726 (C=O, acetate); 1703 (C=O, ketone); 1251, 1236 (C-O); 1262, 1236, 1198 (O-SO₃). ¹H NMR (200 MHz): 0.61 (s, 3 H, 3 x H-18); 0.93 (s, 3 H, 3 x H-19); 2.07 (s, 3 H, OAc); 2.12 (s, 3 H, 3 x H-21); 2.54 (t, 1 H, J = 8.8, H-17); 4.23 (m, 1 H, W = 32, H-3); 4.84 (m, 1 H, H-7). FAB MS: 449 (3%), 427 (2.5%), 405 (2.5%), 381 (5%), 307 (10%), 285 (10%), 263 (10%), 143 (100%). C₂₃H₃₅NaO₇S: Calculated C, 57.72; H, 7.37; S, 6.70. Found C, 50.66; H, 6.85; S, 4.57.

### Example 15

### 3α-(tert-Butyldimethylsilyloxy)-5β-pregnan-7α-yl Acetate

Hydroxyderivative prepared in Example 14 (70 mg, 0.18 mmol) and imidazole (76 mg, 1.1 mmol) were dissolved in N,N-dimethylformamide (1.4 mL). The mixture was cooled to 0 °C, then tert-butyldimethylsilyl chloride was added (84 mg, 0.56 mmol). The reaction mixture was stirred at room temperature. After 2 h, the reaction was diluted with ethyl acetate (50 mL) and washed with solution of citric acid, potassium hydrogen carbonate, and water. The organic layer was dried and evaporated in vacuo. Crystallization form ethyl acetate afforded title compound (50 mg, 55%): m.p. 110-112 °C, [α]_{D}+30.5 (c 0.333, CHCl₃). IR spectrum (CHCl₃): 2955, 2906 ((CH₃)₃C); 1725 (C=O, acetate); 1702 (C=O, ketone); 1254, 1021 (C-O, acetate); 1090, 1076 (C-OSi); 853, 837 ((CH₃)₂Si). ¹H NMR (200 MHz): 0.05 (s, 6 H, (CH₃)₂Si); 0.6 (s, 3 H, 3 × H-18); 0.88 (s, 9 H, (CH₃)₃C); 0.91 (s, 3H, 3 × H-19); 2.04 (s, 3 H, OAc); 2.12 (s, 3 H, 3 × H-21); 2.54 (t, 1 H, J = 8.7, H-17); 3.45 (m, 1 H, W = 32, H-3); 3.87 (q, 1 H, J = 2.4, H-7). FAB MS: 433 (2%, M - 57, (CH₃)₃C), 373 (18%, M - (CH₃)₃C, AcOH), 299 (100%, M - (CH₃)₃(CH₃)₂Si, AcOH), 255 (12%, M - (CH₃)₃(CH₃)₂SiO, AcOH, CH₃CO). C₂₉H₅₀O₄Si: Calculated C, 70.97; H, 10.27. Found C, 70.03; H, 10.44.

### Example 16

### 3α-(tert-Butyldimethylsilyloxy)-7α-hydroxy-5β-pregnan-20-one

A solution of acetate prepared in Example 15 (40 mg, 0.08 mmol) in benzene (5 mL) was refluxed with a solution of potassium hydroxide in ethanol (4 mL, 0.89 M) for 3 h. The mixture was poured into water and extracted with ether (40 mL). The organic layer was washed with water, dried and the solvents were evaporated in vacuo. Preparative thin layer chromatography in a mixture of petroleum ether/acetone (8:2) afforded the title compound (20 mg, 55%): m.p. 131-136 °C, [α]_{D}+47.6 (c 0.29, CHCl₃). IR spectrum (CHCl₃): 3621 (O-H); 1699 (C=O); 1472, 1463, 1385 ((CH₃)₃C); 1361 (Ac, (CH₃)₃C); 1254 ((CH₃)₂Si); 1098,1082 (C-OSi). ¹H NMR (200 MHz): 0.05 (s, 6 H, (CH₃)₂Si); 0.60 (s, 3 H, 3 x H-18); 0.88 (s, 12 H, 3 x H-19 and (CH₃)₃C); 2.12 (s, 3 H, 3 x H-21); 2.53 (t, 1 H, J = 9, H-17); 3.41 (m, 1 H, W = 32.7, H-3); 3.86 (m, 1 H, H-7). FAB MS: 449 (11%, M + 1), 317 (10%, M - (CH₃)₃(CH₃)₂SiO), 299 (26%, M + 1 - (CH₃)₃(CH₃)₂SiO, H₂O), 283 (13%, M + 1 - (CH₃)₃(CH₃)₂SiO, H₂O, O), 255 (14%, M - (CH₃)₃(CH₃)₂SiO, H₂O, CH₃CO). C₂₇H₄₈O₃Si: Calculated C, 72.26; H, 10.70. Found C, 72.20; H, 10.84.

### Example 17

### 3α-(tert-Butyldimethylsilyloxy)-20-oxo-5β-pregnan-7α-yl Nicotinate

Compound from previous Example 16 (240 mg, 0.53 mmol) and 4-dimethylaminopyridine (10 mg, 0.09 mmol) were dissolved in pyridine (10 mL) and the solution was cooled to 0 °C. Nicotinoyl chloride hydrochloride (720 mg, 4.0 mmol) was slowly added. The reaction mixture was stirred at room temperature. After 10 h, it was poured into water and extracted with ethyl acetate (100 mL). Aqueous phase was extracted again with ethyl acetate (100 mL), and the combined organic phases were dried and evaporated. Preparative thin layer chromatography in a mixture of petroleum ether/acetone (9:1) afforded title compound (208 mg, 70%): m.p. 57-59 °C, [α]_{D} +54.9 (c 0.387, CHCl₃). IR spectrum (CHCl₃): 2907 (CH₃, (CH₃)₃(CH₃)₂SiO); 1714 (C=O, nicotinate); 1703 (C=O, ketone); 1286, 1108 (C-O, nicotinate); 1092 (C-OSi). ¹H NMR (400 MHz): 0.05 (s, 6 H, (CH₃)₂Si); 0.63 (s, 3 H, 3 × H-18); 0.77 (s, 9 H, (CH₃)₃C); 0.96 (s, 3 H, 3 × H-19); 2.11 (s, 3 H, 3 × H-21); 2.52 (t, 1 H, J = 9.1, H-17); 3.43 (m, 1 H, W = 32, H-3); 5.21 (q, 1 H, J = 2.8, H-7); 7.44 (ddd, 1 H, J₁ = 7.8, J₂ = 7.8, J₃ = 0.7, H-5, nicotinate); 8.30 (dt, 1 H, J₁ = 8.3, J₂ = 2, H-4, nicotinate); 8.78 (dd, 1 H, J₁ = 4.8, J₂ = 1.7, H-6, nicotinate); 9.27 (dd, 1 H, J₁ = 2, J₂ = 0.7, H-2, nicotinate). FAB MS: 579 (6%, M + Na), 554 (22%, M + 1), 496 (7%, M - (CH₃)₃C), 299 (4%, M - (CH₃)₃(CH₃)₂SiO, OCOC₆H₄N), 255 (79%, M - (CH₃)₃C(CH₃)₂SiO, OCOC₆H₄N, CH₃CO). C₃₃H₅₁O₄Si: Calculated C, 71.56; H, 9.28; N, 2.53. Found C, 70.77; H, 9.32; N, 2.23.

### Example 18

### 3α-Hydroxy-20-oxo-5β-pregnan-7α-yl Nicotinate

A solution of compound prepared in Example 17 (100 mg, 0.18 mmol) in dry THF (10 mL) was cooled to 0 °C and solution of tetrabutylammonium fluoride (1 M in THF, 0.3 mL, 0.3 mmol) was added. The mixture was stirred at room temperature. After 2 days, further tetrabutylammonium fluoride solution (1 M in THF, 0.1 mL, 0.1 mmol) was added and the mixture was stirred for another 3 days. The mixture was extracted with ethyl acetate (100 mL), organic layer was washed solution of citric acid, potassium hydrogen carbonate, water, and dried. Preparative thin layer chromatography (2 plates) in a mixture of petroleum ether/acetone (9:1) afforded title compound (58 mg, 74%): m.p. 70-73 °C (acetone/heptane), [α]_{D} +29.2 (c 0.279, CHCl₃). IR spectrum (CHCl₃): 3613; 3451 (O-H); 1715 (C=O, nicotinate); 1705 (C=O, ketone); 1287 (C-O, nicotinate)'; 1034, 1026 (C-OH); 1592, 1421 (nicotinate). ¹H NMR (400 MHz): 0.64 (s, 3 H, 3 × H-18); 0.98 (s, 3 H, 3 × H-19); 2.12 (s, 3 H, 3 × H-21); 2.54 (t, 1 H, J = 9.2, H-17); 3.49 (m, 1 H, W = 31, H-3); 5.20 (q, 1 H, J = 2.7, H-7); 7.45 (ddd, 1 H, J₁ = 7.8, J₂ = 4.8, J₃ = 0.7, H-5, nicotinate); 8.31 (dt, 1 H, J₁ = 7.8, J₂ = 1.7, H-4, nicotinate); 8.80 (dd, 1 H, J₁ = 4.8, J₂ = 1.7, H-6, nicotinate); 9.26 (dd, 1 H, J₁ = 2, J₂ = 0.7, H-2, nicotinate). FAB MS: 440 (12%, M + 1), 145 (7%), 124 (1.00%), 105 (35%). C₂₇H₃₇NO₄: Calculated C, 73.77; H, 8.48; N, 3.19. Found C, 73.71; H, 8.95; N, 2.67.

### Example 19

### 20-Oxo-5β-pregnane-3α,7α-diyl 3-Sulfate 7-Nicotinate Pyridinium Salt

The mixture of compound prepared, in Example 18 (45 mg, 0.1 mmol) and a sulfur trioxide pyridine complex (90 mg, 0.56 mmol), dried in vacuo (25 °C, 100 Pa) for 1 h, was dissolved in dry chloroform (5 mL) and under argon stirred for 4 h. After standing at -20 °C overnight, the undissolved sulfur trioxide pyridine complex was filtered off and evaporated. the residue was dissolved in methanol (1 mL) and diethylether (4 mL). The mixture was let to stand overnight at -20 °C. The solvents were evaporated to afford title compound (40 mg, 74%) as a foam: [α]_{D} +42.0 (c 0.321, CHCl₃). IR spectrum (CHCl₃): 3139, 2652 (pyridinium); 1714 (C=O, nicotinate); 1703 (C=O, ketone); 1287 (C-O, nicotinate); 1175, 978, 958 (O-SO₃). ¹H NMR (400 MHz): 0.63 (s, 3 H, 3 x H-18); 0.98 (s, 3 H, 3 x H-19); 2.11 (s, 3 H, 3 x H-21); 2.56 (t, 1 H, J = 8.7, H-17); 4.32 (m, 1 H, W = 32, H-3); 5.26 (m, 1 H, H-7); 7.74 (dd, 1 H, J₁ = 7.6, J₂ = 5.3, H-5, nicotinate); 7.82 (m, 2 H, H-3 and H-5, pyridinium); 8.33 (t, 2 H, J =7.8, H-4, pyridinium); 8.60 (d, 1 H, J = 7.6, H-4, nicotinate); 8.88 (m, 2 H, H-2 and H-6, pyridinium); 8.93 (d, 1 H, J = 5, H-6, nicotinate); 9.33 (m, 1 H, H-2, nicotinate). FAB MS: 564 (10%), 542 (20%), 519 (12%, M + 1 pyridinium), 444 (18%), 422 (10%, M + 1 - OSO₃C₅H₆N). HR-MS (+ESI) Calculated for C₃₂H₄₂O₇N₂NaS [M⁺ + Na] 621.2605, found 621.2608. C₃₂H₄₂N₂O₇S: Calculated C, 64.19; H, 7.07; N, 4.68; S, 5.36. Found C, 58.39; H, 6.88; N, 3.70; S, 6.99.

### Example 20

### Biological Activity Assay

### Cell Culture

Primary dissociated hippocampal cultures were prepared from 1- to 2-day-old postnatal rats. Animals were decapitated and the hippocampi dissected. Trypsin digestion, followed by mechanical dissociation, was used to prepare cell suspension. Single cells were plated at a density of 500,000 cells/cm² on 31 mm or 12 mm polylysine-coated glass cover slips. Neuronal cultures were maintained in Neurobasal^{™}-A (Invitrogen, Carlsbad, USA) medium supplemented with glutamine (0.5 mM) and B-27 Serum-Free Supplement (Invitrogen) at 37°C and 5% CO₂.

### Electrophysiology

For the electrophysiological experiments, neurons cultured for 5-10 days were used. Whole-cell voltage-clamp recordings were made with a patch-clamp amplifier (Axopatch 1D; Axon Instruments, Inc., Foster City, USA) after capacitance and series resistance (<10 MΩ) compensation of 80 - 90%. Agonist-induced responses were low-pass filtered at 1 kHz with an 8-pole Bessel filter (Frequency Devices, Haverhill, USA), digitally sampled at 5 kHz and analyzed using pClamp software version 9 (Axon Instruments). Patch pipettes (3-6 MΩ) pulled from borosilicate glass were filled with Cs⁺ based intracellular solution containing (in mM): 125 gluconic acid, 15 CsCl, 5 EGTA, 10 HEPES, 3 MgCl₂, 0.5 CaCl₂, and 2 ATP-Mg salt (pH-adjusted to 7.2 with CsOH). Extracellular solution (ECS) contained (in mM): 160 NaCl, 2.5 KCl, 10 HEPES, 10 D-glucose, 0.2 EDTA and 0.7 CaCl₂ (pH-adjusted to 7.3 with NaOH). Glycine (10 µM) and TTX (0.5 µM) were present in the control and test solutions. Steroids were dissolved in dimethyl sulfoxide (DMSO) to make a fresh stock solution (20 mM) before each experiment. The same concentration of DMSO was maintained in all extracellular solutions. A microprocessor-controlled multibarrel fast perfusion system, with a time constant of solution exchange around cells of ~10 ms, was used to apply test and control solutions.

### Biological Activity

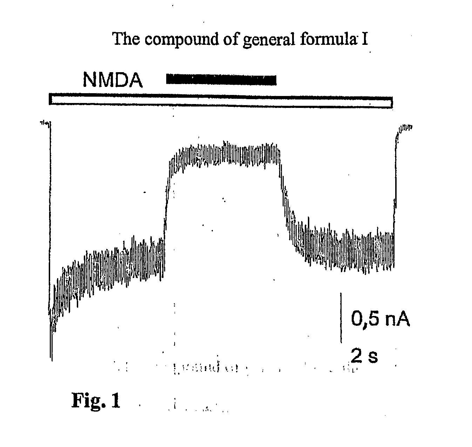

Responses (currents) elicited by 100 µM NMDA were recorded in cultured hippocampal neurons voltage-clamped at a holding potential of - 60 mV. In accordance with previous results, pregnanolone sulfate diminished the amplitude of NMDA-induced currents (Fig. 1). At 100µM pregnanolone sulfate, the mean inhibitory effect was 71.3 ± 5.0% (n = 5). Synthetic analogs of pregnanolone sulfate had also negative modulatory effect on NMDA-induced responses with the mean inhibition induced by 100µM steroid that ranged from 17% (compound from Example 5) to 71 % (5β-pregnenolone sulfate). The relative degree of steroid-induced inhibition was used to estimate IC₅₀. IC₅₀ was calculated from the logistic equation RI =1- (1 / (1 + ([steroid] / IC₅₀)^{h}); where RI is relative degree of steroid induced inhibition and h is the apparent Hill coefficient (1.2). The values of relative inhibition and estimated IC₅₀ values of steroids are listed in following table.

**Table**

| Inhibition of NMDA-induced response in cultured hippocampal neurons by pregnanolone sulfate and its synthetic analogs. | | | | |
|---|---|---|---|---|
| Compound from Example | Inhibition (X) | SD | IC₅₀ | IC₅₀ SD |
| 12 | 21.4 | 2.7 | 299.9 | 40.9 |
| 13 | 34.1 | 10.2 | 185.8 | 61.1 |
| 19 | 46.9 | 9.7 | 116.0 | 36.5 |
| 3 | 17.4 | 4.3 | 383.5 | 93.5 |
| 4 | 35.2 | 2.7 | 167.2 | 16.3 |
| 10 | 37.8 | 4.7 | 153.8 | 25.0 |

The second column shows relative degree of steroid (100 µM) inhibition of current responses induced in cultured hippocampal neurons by fast application of 100 µM NMDA and co-application of 100µM of pregnenolone sulfate.

## Claims

1. Pregnane anionic compounds of general formula I 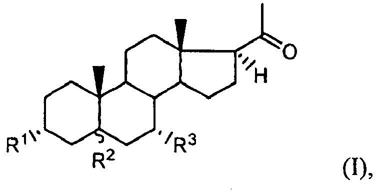 in which
R¹ is the ester group of general formula R⁴O-, which is able to form anion, where R⁴ is HSO₃-, pyridine-SO₃-, HOOC-(CH₂)₂-CO-,
R² is hydrogen atom with the configuration of the alpha or beta and
R³ is ester group of general formula R⁵COO-, where R⁵ is CH₃- or C₅H₄N-, such as acetoxy group or nicotinyloxy group,
and their pharmaceutically acceptable salts.

2. Pregnane anionic compounds of general formula I according to claim 1, which are:
20-Oxo-5α-pregnane-3α,7α-diyl 3-Hemisuccinate 7-Acetate,
20-Oxo-5α-pregnane-3α,7α-diyl 3-Sulfate 7- Acetate Pyridinium Salt,
20-Oxo-5α-pregnane-3α,7α-diyl 3- Sulfate 7-Nicotinate Pyridinium Salt,
20-Oxo-5β-pregnane-3α,7α-diyl 3-Hemissuccinate 7- Acetate,
20-Oxo-5β-pregnane-3α,7α-diyl 3- Sulfate 7- Acetate Pyridinium Salt,
20-Oxo-5β-pregnane-3α,7α-diyl 3- Sulfate 7- Acetate Sodium Salt, and
20-Oxo-5β-pregnane-3α,7α-diyl 3-Sulfate 7-Nicotinate Pyridinium Salt.

3. A method for producing of compounds of general formula **I** according to claim 1 to 2, in which R¹, R² are as given in claim 1 and R³ is CH₃COO-, where the compound of formula **II** 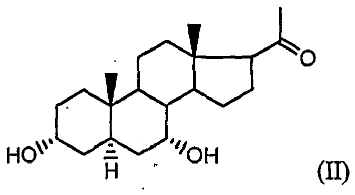 is converted to 3,7-diprotected derivative, which by partial hydrolysis in a suitable solvent such as alcohol, aqueous alcohol, or mixed solvents, advantageously in a
mixture of methanol and benzene, is converted to monoester of formula **IV** 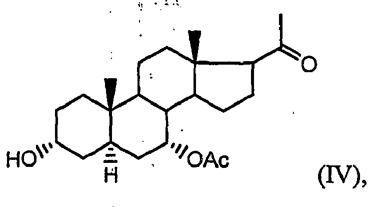 from which by treatment with succinic anhydride in pyridine in the presence of DMAP affords particular hemisuccinate of formula **I**, in which R¹ is hemisuccinate group, R² is a hydrogen atom in the alpha position and R³ is acetoxy group,
or by the treatment with pyridine-sulfur trioxide complex in chloroform affords pyridinium sulfate of formula **I,** in which R¹ is pyridinium sulfate group, R² is a hydrogen atom in the alpha position and R³ is acetoxy group, whereupon the latter compound of formula **I,** in which R¹ is pyridinium sulfate group, R² is a hydrogen atom in the alpha position and R is acetoxy group, on reaction with sodium hydroxide in alcohol, advantageously in methanol, can be converted to compound of formula **I**, in which R¹ is sodium salt of sulfate, R² is hydrogen atom in the alpha position and R³ is acetoxy group.

4. The method according to claim 3, wherein the 3,7-diprotected derivative is diacetate of formula **III**

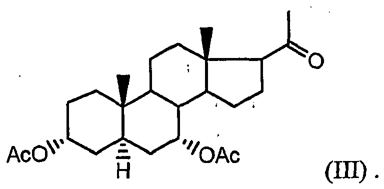

5. The method according to claim 3 or 4, wherein the partial hydrolysis is carried out by treatment with alkali metal hydroxide such as potassium, sodium or lithium hydroxide, or acids such as hydrochloric or perchloric acid.

6. The method according to any of claims 3 to 5, wherein the appropriate solvent is alcohol, aqueous alcohol or mixture of solvents advantageously a mixture of methanol and benzene.

7. The method according, to any of claims 3 to 6, wherein the mixture of solvents is the mixture of methanol and benzene.

8. The method according to any of claims 3 to 7, wherein the reaction with alkali metal hydroxide is carried out by treatment with potassium hydroxide in alcohol.

9. The method according to any of claims 3 to 8, wherein alcohol is methanol.

10. A method for producing of compound of general formula **I** according to claim 1 and 2, wherein R¹ is pyridinium sulfate, R² is hydrogen atom in the alpha position and R³ is nicotinoyloxy group, **characterized by** the fact that the compound of formula **IV** 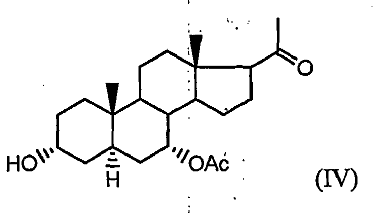 is converted by treatment with *tert*-butyldimethylsilyl chloride and imidazole in a suitable solvent to tert-butyldimethylsilyl derivative of formula **V** 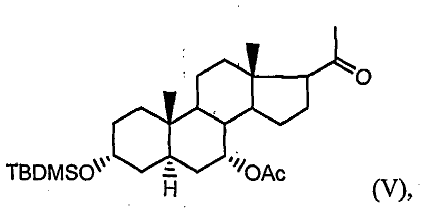 which on saponification of acetate group in position 7 at increased temperature provided compound of formula **VI** 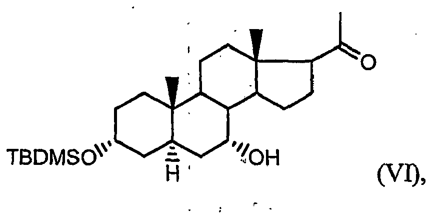 which is esterified on treatment with nicotinoyl chloride in pyridine in the presence of DMAP to produce nicotinyloxy derivative of formula **VII** 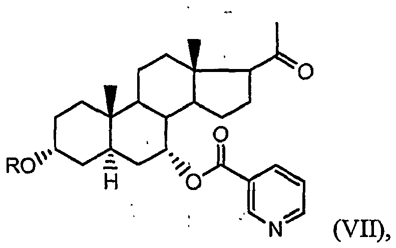 where R is TBDMS, and this derivative on reaction with p-toluenesulfonic acid in methanol afforded compound of formula **VII**, where R is a hydrogen, which then is treated with pyridine-sulfur trioxide complex in a suitable inert solvent.

11. The method according to claim 10, wherein the suitable solvent is DMF.

12. The method according to claim 10 or 11, wherein saponification is saponification with potassium hydroxide in a mixture of ethanol and benzene.

13. The method according to claim 10, wherein convenient inert solvent is chloroform.

14. The method for producing of compound of general formula I according to claim 1 and 2, wherein R² is hydrogen in the beta position, comprising reacting of the compound of formula **VIII** 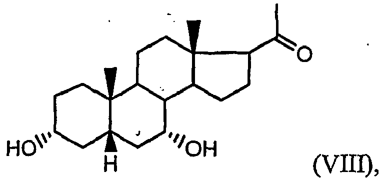 which differs from a compound of formula II only in configuration of the hydrogen atoms in position 5, in the same manner as described for compound II in claims 3 to 13.

15. The method for producing compound of general formula I as defined in claim 1, 2 or 14, wherein from the compound of formula II or VIII, respectively, according to claims 3 to 13 and 14, respectively, the following compounds of general formula **I** are produced:
20-Oxo-5α-pregnane-3α,7α-diyl 3-Hemisuccinate 7-Acetate,
20-Oxo-5α-pregnane-3α,7α-diyl 3-Sulfate 7- Acetate Pyridinium Salt,
20-Oxo-5α-pregnane-3α,7α-diyl 3- Sulfate 7- Acetate Sodium Salt,
20-Oxo-5α-pregnane-3α,7α-diyl 3- Sulfate 7-Nicotinate Pyridinium Salt,
20-Oxo-5β-pregnane-3α,7α-diyl 3- Hemisuccinate 7- Acetate,
20-Oxo-5β-pregnane-3α,7α-diyl 3- Sulfate 7- Acetate Pyridinium Salt,
20-Oxo-5β-pregnane-3α,7α-diyl 3-Sulfate 7- Acetate Sodium Salt, and
20-Oxo-5β-pregnane-3α,7α-diyl 3- Sulfate 7-Nicotinate Pyridinium Salt.

16. Use of compounds of general formula **I** according to claims 1 to 2 for production of pharmaceuticals for the treatment of neurological and psychiatric diseases and conditions associated with excessive activation of NMDA receptors such as neuroprotective agents against excitotoxic damage of the central nervous system (CNS), conditions associated with excessive or NMDA-subtype glutamate receptors or, where this type of receptor is involved in the development or course of several mental and neurological diseases, in particular concerning the traumatic and hypoxic damage of nervous tissue in the central nervous system diseases, such as Alzheimer's, Huntington's and Parkinson's disease, also in cognitive disorders in aging tardive dyskinesia, amyotrophic lateral sclerosis, olivopontocerebellar degeneration, neurological problems associated with AIDS infection, allergic encephalomyelitis, and for medication of epilepsy, anxiety, depression, schizophrenia, chronic pain and drug addiction.

17. Pharmaceuticals comprising an effective amount of compounds of general formula **I** according to claim 1 and 2 and, and optionally other adjuvans or drugs.

18. Use pharmaceuticals according to claim 17 for production of drugs for treatment of neurological and psychiatric diseases and conditions associated with excessive activation of NMDA receptors such as neuroprotective agents against excitotoxic damage of the central nervous system (CNS), conditions associated with excessive activation of NMDA-subtype of glutamate receptors or, where the type of receptor is involved in the development or course of several neurological and mental diseases, in particular concerning the traumatic and hypoxic damage to nervous tissue in the central nervous system diseases such as Alzheimer's, Huntington's and Parkinson's disease, as well as in cognitive disorders in aging, tardive dyskinesia, amyotrophic lateral sclerosis, olivopontocerebellar degeneration, neurological problems associated with AIDS infection, allergic encephalomyelitis, epilepsy, anxiety, depression, schizophrenia, chronic pain and drug addiction.

## Patentansprüche

1. Von Pregnan abgeleitete anionische Verbindungen der allgemeinen Formel **I** 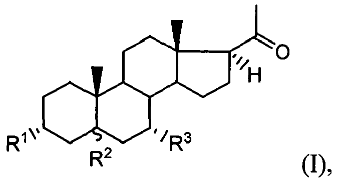 in denen
R¹ eine Estergruppe der Formel R⁴O- ist, die Anionen bilden kann, in der R⁴ HSO₃⁻, Pyridin-SO₃⁻, HOOC-(CH₂)₂-CO⁻ ist,
R² ein Wasserstoffatom ist, das entweder alpha- oder beta-Konfiguration besitzt,
R³ eine Estergruppe der Formel R⁵COO- ist, in der R⁵ CH₃- or C₅H₄N- ist, und zwar eine Acetoxygruppe oder Nicotinoyloxygruppe,
und deren pharmazeutisch akzeptable Salze.

2. Von Pregnan abgeleitete anionische Verbindungen der allgemeinen Formel **I** gemäß Anspruch 1 sind:
20-Oxo-5α-pregnan-3α,7α-diyl-3-hemisuccinat-7-acetat,
Pyridinium-20-oxo-5α-pregnan-3α,7α-diyl-3-sulfat-7-acetat,
Pyridinium-20-oxo-5α-pregnan-3α,7α-diyl-3-sulfat-7-nicotinat,
20-Oxo-5β-pregnan-3α,7α-diyl-3-hemisuccinat-7-acetat,
Pyridinium-20-oxo-5β-pregnan-3α,7α-diyl-3-sulfat-7-acetat,
Natrium-20-oxo-5β-pregnan-3α,7α-diyl-3-sulfat-7-acetat und
Pyridinium-20-oxo-5β-pregnan-3α,7α-diyl-3-sulfat-7-nicotinat.

3. Verfahren zur Herstellung von Verbindungen der allgemeinen Formel **I** gemäß den Ansprüchen 1 und 2, in denen R¹, R² wie in Anspruch 1 beschrieben sind, und R³ CH₃COO⁻ ist, indem die Verbindung der Formel **II** 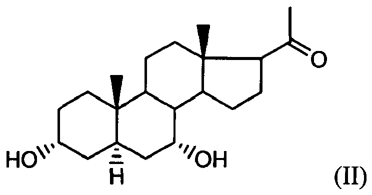 zu einem 3,7-digeschützten Derivat umgewandelt wird, das durch partielle Hydrolyse in einem geignetem Lösungsmittel, wie Alkoholen, wässrigen Alkoholen oder Lösungsmittelgemischen, vorteilhaft in einem Gemisch aus Methanol und Benzol, zum Monoester der Formel **IV** umgesetzt wird, 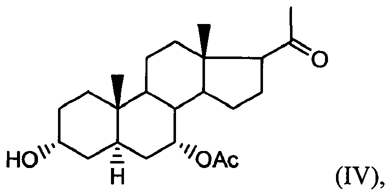 der durch Behandlung mit Bernsteinsäureanhydrid in Pyridin in Anwesenheit von DMAP den Bernsteinsäurehalbester der Formel **I** liefert, in der R¹ die Bemsteinsäurehalbestergruppe, R² ein alpha-orientiertes Wasserstoffatom und R³ eine Acetoxygruppe ist, oder durch Behandlung mit dem Schwefeltrioxid-Pyridin-Komplex in Chloroform ein Pyridiniumsulfate der Formel **I** ergibt, in dem R¹ die Pyridiniumsulfatgruppe, R² ein alpha-orientiertes Wasserstoffatom und R³ eine Acetoxygruppe ist, woraufhin die letztere Verbindung der Formel **I,** in der R¹ die Pyridiniumsulfatgruppe, R² ein alpha-orientiertes Wasserstoffatom und R³ eine Acetoxygruppe ist, bei Behandlung mit Natriumhydroxid in einem Alkohol, vorteilhaft in Methanol, zu Verbindung **I** umgesetzt wird, in der R¹ Natriumsulfat, R² ein alpha-orientiertes Wasserstoffatom und R³ eine Acetoxygruppe ist.

4. Verfahren gemäß Anspruch 3, worin das 3,7-Derivat ein Diacetat der Formel **III** ist

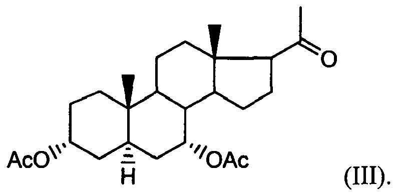

5. Verfahren gemäß den Ansprüchen 3 oder 4, in dem die partielle Hydrolyse durch Behandlung mit Alkalihydroxiden wie zum Beispiel Kalium-, Natrium- oder Lithiumhydroxid oder mit Säuren wie zum Beispiel Salzsaüre oder Perchlorsäure durchgeführt wird.

6. Verfahren gemäß jedes der Ansprüche 3 bis 5, in dem das geeignete Lösungsmittel ein Alkohol, ein wässriger Alkohol oder ein Gemisch aus Lösungsmitteln, vorteilhaft ein Gemisch aus Methanol und Benzol, ist.

7. Verfahren gemäß jedes der Ansprüche 3 bis 6, in dem das Gemisch der Lösungsmittel ein Gemisch aus Methanol und Benzol ist.

8. Verfahren gemäß jedes der Ansprüche 3 bis 7, in dem die Reaktion mit Alkalihydroxiden mit Kaliumhydroxid in einem Alkohol durchgeführt wird.

9. Verfahren gemäß jedes der Ansprüche 3 bis 8, in dem der Alkohol Methanol ist.

10. Ein Verfahren zur Herstellung von Verbindungen der Formel **I** gemäß Ansprüchen 1 and 2, worin R¹ Pyridiniumsulfat, R² ein alpha-orientiertes Wasserstoffatom und R³ die Nicotinoyloxygruppe ist, das **dadurch gekennzeichnet ist, dass** die Verbindung der Formel **IV** 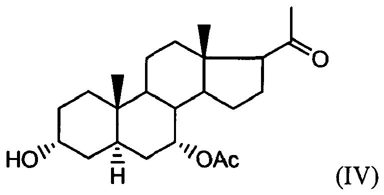 durch Behandlung mit tert-Butyldimethylsilylchlorid und Imidazol in einem geigneten Lösungsmittel zum tert-Butyldimethylsilyl-Derivat der Formel **V** umgesetzt wird 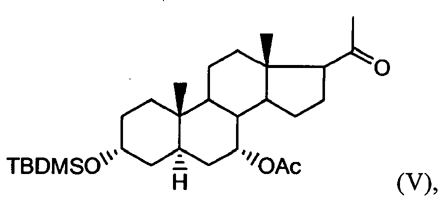 das durch Verseifung der Acetatgruppe in der 7-Position bei erhöhter Temperatur die Verbindung der Formel **VI** liefert, 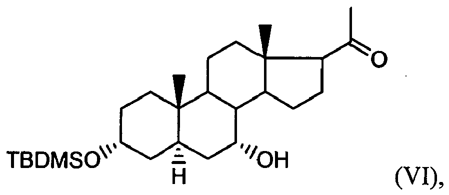 die durch Veresterung mit Nicotinsäurechlorid in Pyridin in Anwesenheit von DMAP das Nicotinoyloxyderivat der Formel **VII** ergibt, 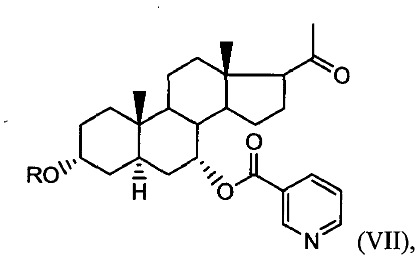 in der R TBDMS ist, und die Behandlung dieses Derivats mit *p-*Toluensulfonsäure in Methanol die Verbindung der Formel **VII** ergibt, in der R ein Wasserstoffatom ist, die dann mit dem Pyridin-Schwefeltrioxid-Komplex in einem geigneten inerten Lösungsmittel behandelt wird.

11. Verfahren gemäß Anspruch 10, worin das geeignete Lösungsmittel DMF ist.

12. Verfahren gemäß Ansprüchen 10 oder 11, in dem die Verseifung mit Kaliumhydroxid in einem Gemisch aus Ethanol und Benzol durchgeführt wird.

13. Verfahren gemäß Anspruch 10, worin das geeignete inerte Lösungsmittel Chloroform ist.

14. Verfahren zur Herstellung von Verbindungen der allgemeinen Formel **I** gemäß Ansprüchen 1 und 2, in denen R² ein beta-orientiertes Wasserstoffatom ist, auf der Basis der Umwandlungen der Verbindung der Formel **VIII,** 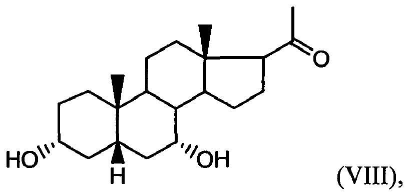 die sich von der Verbindung der Formel **II** nur in der Konfiguration des Wassertoffatoms in der 5-Position unterscheidet, in der gleichen Weise, wie sie für Verbindung **II** gemäß Ansprüchen 3 bis 13 beschrieben wurden.

15. Verfahren zur Herstellung von Verbindungen der allgemeinen Formel **I,** wie in Ansprüchen 1, 2 oder 14 definiert, nach dem folgende Verbindungen der Formel **I** aus den Verbindungen der Formel **II** oder **VIII** gemäß Ansprüchen 3 bis 13 und 14 hergestellt werden:
20-Oxo-5α-pregnan-3α,7α-diyl-3-hemisuccinat-7-acetat,
Pyridinium-20-oxo-5α-pregnan-3α,7α-diyl-3-sulfat-7-acetat,
Natrium-20-oxo-5α-pregnan-3α,7α-diyl-3-sulfat 7-acetat,
Pyridinium-20-oxo-5α-pregnan-3α,7α-diyl-3-sulfat 7-nicotinat,
20-Oxo-5β-pregnan-3α,7α-diyl-3-hemisuccinat-7-acetat,
Pyridinium-20-oxo-5β-pregnan-3α,7α-diyl-3-sulfat-7-acetat,
Natrium-20-oxo-5β-pregnan-3α,7α-diyl 3-sulfat-7-acetat, und
Pyridinium-20-oxo-5β-pregnan-3α,7α-diyl-3-sulfat-7-nicotinat.

16. Die Anwendung von Verbindungen der allgemeinen Formel **I** gemäß Ansprüchen 1 und 2 zur Herstellung von Arzneimitteln für die Behandlung von neurologischen und psychiatrischen Krankheiten und Zuständen, die in Zusammenhang mit der exzessiven Aktivierung von NMDA-Rezeptoren stehen, wie zum Beispiel als neuroprotektive Agenzien gegen die excitotoxische Schädigung des Zentralnervensystems (ZNS), Zustände, die in Zusammenhang mit der exzessiven Aktivierung von Glutamatrezeptoren vom NMDA-Subtyp, oder wo der Rezeptortyp an der Entwicklung und dem Verlauf einer Reihe von von mentalen und neurologischen Erkrankungen beteiligt ist, insbesondere bezüglich der traumatischen und hypoxischen Schädigung des Nervengewebes während Erkrankungen des Zentralnervensystems, wie zum Beispiel Alzheimers, Huntingtons und Parkinsons Erkrankungen, ebenso bei kognitiven Alterserkrankungen, tardiver Dyskinesia, amyotropher Lateralsklerose, olivopontocerebellarer Degeneration, neurologischen Problemen, die mit der AIDS-Infektion assoziiert sind, allergischer Encephalomyelitis, und für Arzneimittel gegen Epilepsie, Angst, Depression, Schizophrenie, chronischem Schmerz und Drogensucht.

17. Arzneimittel, die eine wirksame Menge von Verbindungen der allgemeinen Formel **I** gemäß Ansprüchen 1 und 2 und optional andere Hilfsmittel oder Wirkstoffe enthalten.

18. Die Anwendung von Arzneimitteln gemäß Anspruch 17 zur Herstellung von Medikamenten für die Behandlung von neurologischen und psychiatrischen Krankheiten und Zuständen, die im Zusammenhang mit der exzessiven Aktivierung von NMDA-Rezeptoren stehen, wie zum Beispiel als neuroprotektive Agenzien gegen die excitotoxische Schädigung des Zentralnervensystems (ZNS), Zustände, die im Zusammenhang mit der exzessiven Aktivierung von Glutamatrezeptoren von NMDA-Subtyp, oder wo der Rezeptortyp an der Entwicklung und dem Verlauf einer Reihe von mentalen und neurologischen Erkrankungen beteiligt ist, insbesondere bezüglich der traumatischen und hypoxischen Schädigung des Nervengewebes während Erkrankungen des Zentralnervensystems, wie zum Beispiel im Alzheimers, Huntingtons und Parkinsons Erkrankungen, ebenso bei kognitiven Alterserkrankungen, tardiver Dyskinesia, amyotropher Lateralsklerose, olivopontocerebellarer Degeneration, neurologischen Problemen, die mit der AIDS-Infektion assoziiert sind, allergischer Encephalomyelitis, Epilepsie, Angst, Depression, Schizophrenie, chronischem Schmerz und Drogensucht.

## Revendications

1. Des composés anioniques de prégnane de formule générale **I** 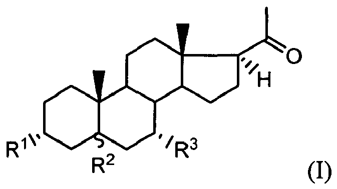 dans lequel
R¹ représente le groupe ester de formule générale R⁴O-, qui est capable de former un anion, où R⁴ est HSO₃-, pyridine-SO₃-, HOOC-(CH₂)₂-CO-,
R² représente un atome d'hydrogène, avec la configuration en alpha ou bêta, et
R³ est un groupe ester de formule générale R⁵COO-, où R⁵ est CH₃ ou C₅H₄N-, tel que le groupe acétoxy ou le groupe nicotinyloxy,
et leurs sels pharmaceutiquement acceptables.

2. Composés anioniques de pregnane de formule générale I selon la revendication 1, qui sont:
7-acétate de 20-oxo-5α-prégnane-3α,7α-diyle 3-hémisuccinate,
7 -acétate de sel de pyridinium de 20-oxo-5α-prégnane-3α,7α-diyle 3-sulfate,
7-Nicotinate sel de pyridinium de 20-oxo-5α-prégnane-3 α,7α-diyle 3-sulfate,
7-acétate de 20-oxo-5β-prégnane-3α,7α-diyle 3-hemissuccinate,
7- acétate de sel de pyridinium de 20-oxo-5β-prégnane-3α,7α-diyle 3-sulfate,
7- acétate de sel de sodium de 20-oxo-5β-prégnane-3α,7α-diyle 3-sulfate, et
7-nicotinate sel de pyridinium de 20-oxo-5β-prégnane- 3α,7α-diyle 3-sulfate.

3. Procédé de production de composés de formule générale I selon la revendication 1 à 2, dans laquelle R¹, R² sont tels que mentionnés dans la revendication 1 et R³ est CH₃COO-, où le composé de formule II 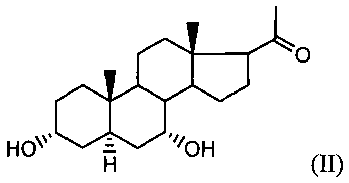 est transformé en dérivé 3,7-diprotégé, qui par une hydrolyse partielle dans un solvant approprié tel qu'un alcool, un alcool aqueux, ou un mélange de solvants, avantageusement dans un mélange de méthanol et de benzène, est converti en mono-ester de formule IV 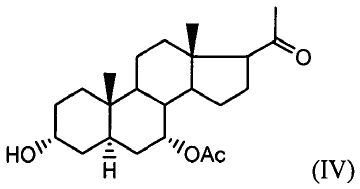 à partir duquel, par traitement avec de l'anhydride succinique dans la pyridine en présence de DMAP donne l'hémisuccinate particulier de formule I, dans laquelle R¹ est un groupe hémisuccinate, R² est un atome d'hydrogène en position alpha et R³ est un groupe acétoxy,
ou par le traitement avec un complexe pyridine-trioxyde de soufre dans le chloroforme donne de pyridinium du sulfate de formule I, dans laquelle R¹ est de pyridinium groupe sulfate, R² est un atome d'hydrogène en position alpha et R³ est un groupe acétoxy, après quoi ce dernier composé de formule I, dans laquelle R¹ est un groupe pyridinium de sulfate, R² est un atome d'hydrogène en position alpha et R³ est un groupe acétoxy, par réaction avec de l'hydroxyde de sodium dans l'alcool, avantageusement dans du méthanol, peut être converti en composé de formule I, dans laquelle R¹ est le sel de sodium de sulfate, R² est un atome d'hydrogène en position alpha et R³ est un groupe acétoxy.

4. Procédé selon la revendication 3, dans lequel le dérivé 3,7-diprotégé est le diacétate de formule III

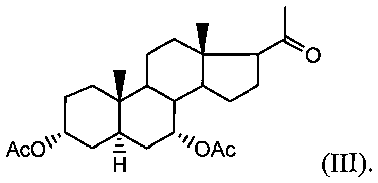

5. Procédé selon la revendication 3 ou 4, dans lequel l'hydrolyse partielle est effectuée par traitement avec l'hydroxyde de métal alcalin tel que l'hydroxyde de potassium, de sodium ou de lithium, ou des acides tels que l'acide chlorhydrique ou perchlorique.

6. Procédé selon l'une quelconque des revendications 3 à 5, dans lequel le solvant approprié est un alcool, un alcool aqueux ou un mélange de solvants, avantageusement un mélange de méthanol et de benzène.

7. Procédé selon l'une quelconque des revendications 3 à 6, dans lequel le mélange de solvants est le mélange de méthanol et de benzène.

8. Procédé selon l'une quelconque des revendications 3 à 7, dans lequel la réaction avec de l'hydroxyde se fait par traitement avec de l'hydroxyde de potassium dans un alcool.

9. Procédé selon l'une quelconque des revendications 3 à 8, dans lequel l'alcool est le méthanol.

10. Procédé de production d'un composé de formule générale I selon la revendication 1 et 2, dans lequel R¹ est un sulfate de pyridinium, R² est un atome d'hydrogène en position alpha et R³ est un groupe nicotinoyloxy, **caractérisé par le fait que** le composé de formule IV 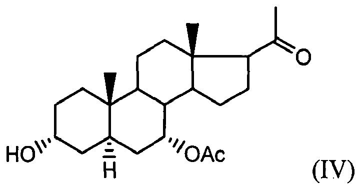 est converti par traitement avec du chlorure de tert-butyldiméthylsilyle et de l'imidazole dans un solvant approprié en un dérivé de tert-butyldiméthylsilyle de formule V 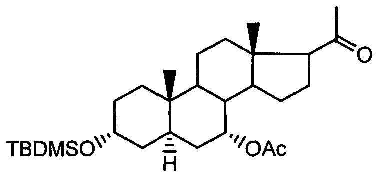 qui par saponification de groupe acétate en position 7 à température élevée fournit le composé de formule VI 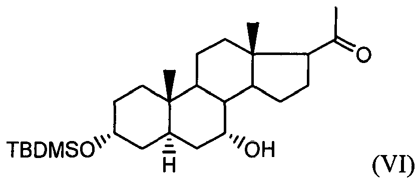 qui est estérifié par de traitement avec du chlorure d'acide nicotinique dans la pyridine en présence de DMAP pour produire le dérivé de formule VII nicotinyloxy 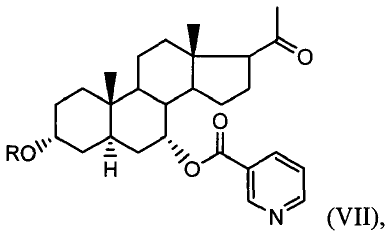 où R est TBDMS, et ce dérivé par une réaction avec l'acide *p*-toluène-sulfonique dans du méthanol a donné le composé de formule VII, où R est un atome d'hydrogène, qui est ensuite traité avec un complexe pyridine-trioxyde de soufre dans un solvant inerte approprié.

11. Procédé selon la revendication 10, dans lequel le solvant approprié est le DMF.

12. Procédé selon la revendication 10 ou 11, dans lequel la saponification est une saponification avec de l'hydroxyde de potassium dans un mélange d'éthanol et de benzène.

13. Procédé selon la revendication 10, dans lequel le solvant inerte est le chloroforme.

14. Procédé de production d'un composé de formule générale I selon la revendication 1 et 2, dans lequel R² est un atome d'hydrogène en position bêta, comprenant la réaction du composé de formule VIII 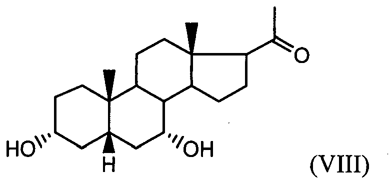 qui diffère d'un composé de formule II que dans la configuration des atomes d'hydrogène en position 5, de la même manière que celle décrite pour le composé II dans les revendications 3 à 13.

15. Procédé pour produire le composé de formule générale I tel que défini dans la revendication 1, 2 ou 14, dans lequel, à partir du composé de formule II ou VIII, respectivement , selon les revendications 3 à 13 et 14, respectivement, les composés suivants de formule générale I sont produits:
7-acétate de 20-oxo-5α-prégnane-3α,7α-diyle 3-hémisuccinate,
7 -acétate de sel de pyridinium de 20-oxo-5α-prégnane-3α,7α-diyle 3-sulfate,
7-Nicotinate sel de pyridinium de 20-oxo-5α-prégnane-3 α,7α-diyle 3-sulfate,
7-acétate de 20-oxo-5β-prégnane-3 α,7α-diyle 3-hemissuccinate,
7- acétate de sel de pyridinium de 20-oxo-5β-prégnane-3α,7α-diyle 3-sulfate,
7- acétate de sel de sodium de 20-oxo-5β-prégnane-3α,7α-diyle 3-sulfate, et
7-nicotinate sel de pyridinium de 20-oxo-5β-prégnane- 3α,7α-diyle 3-sulfate.

16. Utilisation de composés de formule générale I selon les revendications 1 à 2 pour la production de produits pharmaceutiques pour le traitement des maladies et affections neurologiques et psychiatriques associées à une activation excessive des récepteurs de NMDA, comme des agents neuroprotecteurs contre les dommages excitotoxiques du système nerveux central (SNC), conditions associées à l'activation excessive des récepteurs sous-type du glutamate NMDA ou, lorsque ce type de récepteur est impliqué dans l'apparition ou l'évolution de plusieurs maladies mentales et neurologiques, en particulier en ce qui concerne les dommages traumatiques et hypoxie des tissus nerveux dans les maladies du système nerveux central , telles que la maladie d'Alzheimer, la chorée de Huntington et la maladie de Parkinson, également dans les troubles cognitifs liés au vieillissement, la dyskinésie tardive, la sclérose latérale amyotrophique, la dégénérescence olivopontocérébelleuse, problèmes neurologiques associés à l'infection du SIDA, l'encéphalomyélite allergique, et pour les médicaments de l'épilepsie, l'anxiété, la dépression, la schizophrénie, la douleur chronique et la toxicomanie.

17. Produits pharmaceutiques comprenant une quantité efficace de composés de formule générale 1 selon la revendication 1 et 2, et éventuellement d'autres adjuvants ou des médicaments.

18. Utilisation des produits pharmaceutiques selon la revendication 17 pour la production de médicaments pour le traitement de maladies et d'affections neurologiques et psychiatriques associées à une activation excessive des récepteurs de NMDA, comme des agents neuroprotecteurs contre les dommages excitotoxiques du système nerveux central (SNC), d'affections associées à une activation excessive du NMDA sous-type de récepteurs du glutamate, ou, si le type de récepteur est impliqué dans l'apparition ou l'évolution de plusieurs maladies neurologiques et mentales, en particulier en ce qui concerne les dommages traumatiques et hypoxie du tissu nerveux dans les maladies du système nerveux central comme la maladie d'Alzheimer, la chorée de Huntington et la maladie de Parkinson, ainsi que dans les troubles cognitifs liés au vieillissement, la dyskinésie tardive, la sclérose latérale amyotrophique, la dégénérescence olivopontocérébelleuse, les problèmes neurologiques associés à l'infection du SIDA, l'encéphalomyélite allergique, l'épilepsie, l'anxiété, la dépression, la schizophrénie, la douleur chronique et la toxicomanie.
